# EUROPEAN PATENT SPECIFICATION

(11) **EP 0 468 327 B1**
(45) Date of publication and mention of the grant of the patent: **20.11.1996**
(21) Application number: 91111733.1
(22) Date of filing: 15.07.1991
(51) Int. Cl.: A61K 38/00, A61K 9/127

(54) **Oral pharmaceutical compositions containing hirudin**
Hirudin enthaltende orale Arzneimittel
Compositions pharmaceutiques contenant de l'hirudine

(30) Priority: 23.07.1990 IT 2102190
(43) Date of publication of application: 29.01.1992
(73) Proprietor: IKETON FARMACEUTICI S.R.L., I-20090 Segrate (Milano) (IT)
(72) Inventor: Niada, Riccardo, I-20090 Segrate (Milano) (IT); Butti, Adriano, I-20090 Segrate (Milano) (IT)
(74) Representative: Minoja, Fabrizio

(56) References cited:
- EP-A- 0 333 356
- EP-A- 0 372 670
- WO-A-80/01456
- FR-M- 3 792
- US-A- 4 348 384

## Description

The present invention relates to oral pharmaceutical compositions containing recombinant hirudin as the active ingredient.

The invention also relates to the use of recombinant hirudin for the preparation of a medicament to be orally administered for the treatment of both phlebo- and arterious thrombosis.

Native hirudin is a polypeptide having M.W. 7000, which is formed by 65 amino acids, and it has high and specific antithrombin and antithrombotic activity.

Hirudin is extracted from leeches according to Markwardt's method (Z. Phys. U. Chem. 388, 147, 1957), and it has anticoagulant and hemorragic activities (increase in bleeding time).

Biotechnology and Pharmacology researches allowed the preparation and characterization of recombinant hirudin, which differs from the native one, for the lack of a sulfate group on 63-Tyr in its amino acidic sequence and for some distinctive pharmacological features.

Recombinant hirudin does not modify coagulation parameters neither in vitro nor in vivo therefore it is free of anticoagulant activity; moreover it is free of haemorragic activity, since the bleeding time does not increase in rat and rabbit.

Antithrombotic activity is the main feature of recombinant hirudin, as it prevents or inhibits thrombus formation in several animal models (Thromb. Haemostas. 47, 3, 226-229, 1982; Thromb. Res. 43, 613-620, 1986; Pharmazie 43, 202-207, 1988).

Both antithrombotic activity and the lack of anticoagulant and bleeding activities can be evidenced by administering recombinant hirudin either by the intravenous or by the subcutaneous routes.

Pharmacokinetic studies showed that half-life time is about 60 minutes in intravenously administered dogs, whereas the effect is longer lasting (4-5 hours) by subcutaneous administration, with an antithrombinic activity peak at 90-120 minutes.

Only French BSM 3,792 (Special Medicament Patent) mentions the possibility to administer native hirudin by the oral route, but it should be considered that in said patent hirudin was defined as a crude extract from leeches (Hirudo medicinalis), therefore no reliable conclusions can be drawn about the effective oral absorption of native hirudin, and even less of the recombinant one, this latter having different pharmacological characteristics. Probably, the anticoagulant activity disclosed in BSM n. 3,792 was actually due to other components which were present in the used crude extract. This assumption would confirm what stated by other Authors (XI International Congress on Thrombosis and Haemostasis - Brussels 6-July 10, 1987, Abs. n. 280), who showed the results obtained by oral administration of leech saliva in rats, achieving an antithrombotic activity, which was lower than the one obtained by intravenous administration and that increased after repeated administrations. According to these Authors, such an antithrombotic effect cannot be ascribed to hirudin antithrombin activity Leech saliva contains not only hirudin, but also other proteins like eglin, hyaluronidase, collagenase and apyrase. None of the above proteins was stated to be an anticoagulant, including eglin, which showed no antithrombotic effect, the most evident activity of eglin being the inhibition of leukocyte elastase and of catepsin.

The most recent scientific and patent literature agrees in considering parenteral administration the only and exclusively effective administration route for native hirudin or derivatives or fragments thereof, obtained either by synthesis or by recombinant DNA techniques, so confirming the results already obtained by Markwardt et al. (Biomed. Biochim. Acta 46, 4, 237-244, 1987), who found no pharmacological activities after intraduodenal administration of hirudin in the dog.

On the contrary, it has now been found that recombinant hirudin can effectively be administered by the oral route to give rise to a dose-dependent and reproducible antithrombotic activity.

Therefore, the invention provides oral pharmaceutical compositions containing recombinant hirudin as the active ingredient. Said compositions can be prepared according to conventional methods, like those described, for instance, in "Remington's Pharmaceutical Sciences Handbook", Mack Pub. Co., N.Y., USA.

Examples of suitable formulations for the oral administration include tablets, soft or hard gelatin capsules, granulates, powders, instant solutions or suspensions, and the like.

Gastro-resistant forms, particularly those obtained by microgranulation, can advantageously be used in the present invention. Typically, unit doses of hirudin will contain from 1 to 500 mg, to be administered one or more times a day.

According to a particularly preferred embodiment of the invention, liposomial formulations are provided, which can be prepared by incorporating hirudin in egg lecithin in the presence of stearyl amine or ethyl phosphate and by subjecting the mixture to sonication or phase reverse evaporation techniques. Such formulations allow to reduce by 5-10 times the active doses for antithrombotic effect.

The results of pharmacological tests, carried out by oral administration of recombinant hirudin, are hereinafter reported.

### EXAMPLE 1

Activity of orally administered hirudin in stasis-induced thrombosis in rabbit jugular vein.

### Recombinant hirudin under test had a specific activity of 10,000 ATU/mg. New Zealand male rabbits weighing 2.5-2.7 kg were used, fasted for 24 hours, with water ad libitum.

Anesthesia was induced with ketamin (70 mg/kg i.m.) and xylazine (20 mg/kg i.m.). Right jugular vein was isolated for 2 cm lenght; a 3 cm cotton thread was inserted into the jugular vein through the facial vein. Two hemostats were applied upstream and downstream the thread and left in situ for 30 minutes, then they were removed and the thrombus was allowed under free flow for 90 minutes. The thrombus was induced by stasis and by the thread.

After 90 minutes, the thread, with thrombus attached, was removed, dried (80°C, 1 hour) and weighed. Recombinant hirudin dissolved in water was administered at three different doses, in a 1 ml/kg volume, by the oral route (gastric probe), 60 minutes before inducing thrombus formation in the rabbit jugular vein. Control animals received 1 ml/kg water, orally.

Blind tests were carried out; each of them including 4 rabbits: 1 control rabbit and 3 rabbits treated with hirudin at three different doses (see results below). Each test was repeated 10 times.

Results were evaluated by analysis of variance, Dunnet's test, regression; ED₅₀ was calculated.

The results are summarized in Table I.

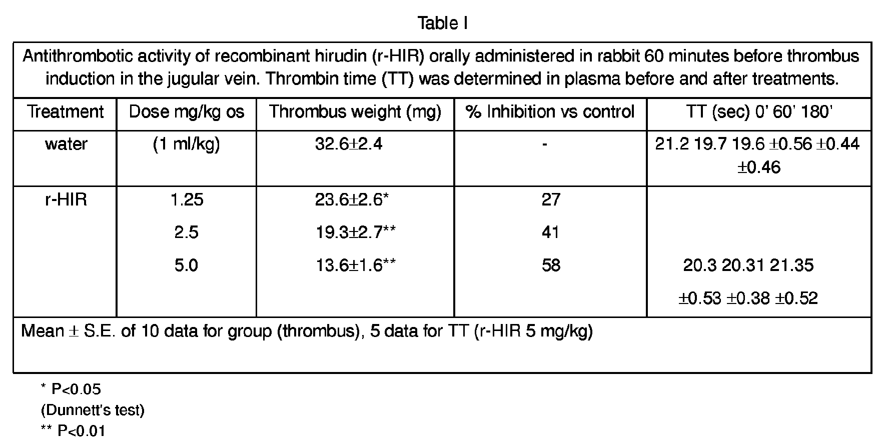

Orally administered hirudin in the rabbit, under the described test conditions, showed surprisingly antithrombotic activity. Hirudin was administered at doses of 1.25-2.5-5 mg/kg and the effects were statistically significant and dose-dependent. ED₅₀ was 3.5 mg/kg.

interestingly hirudin at its highest dose (5 mg/kg os), was found to inhibit thrombosis by 58%, but not to induce modifications of thrombin time, which was determined ex vivo 60 and 180 minutes after the treatment.

### EXAMPLE 2

Activity of orally administered recombinant hirudin (10,000 ATU/mg) in copper-coil thrombosis in rat.

CD male rats (Charles River) weighing 250-260 g, fasted for 24 hours, were used. Thrombosis was induced during pentobarbital anaesthesia (60 mg/kg i.p.) by inserting a copper-coil in the inferior vena cava through the left iliac vein and in caudal position respect to the left renal vein. Thrombi were removed 1 hour after the copper coil insertion, kept at 80°C for 1 hour and weighed.

In some preliminar tests, hirudin (recombinant hirudin, lot F 1-3 1/88) was orally administered in rat at the dose of 2.5 mg/kg (1 ml/kg) 0.5-1-2-4 hours before inducing thrombosis, to find out the maximum response time to the drug. According to the obtained results, antithrombotic activity of hirudin was evaluated at 0.375, 0.75, 1.5, 3 mg/kg (1 ml/kg) doses orally administered four hours before inducing thrombosis. Calcium heparin (HEP/5) was tested under the same experimental conditions.

Results are reported in Table II.

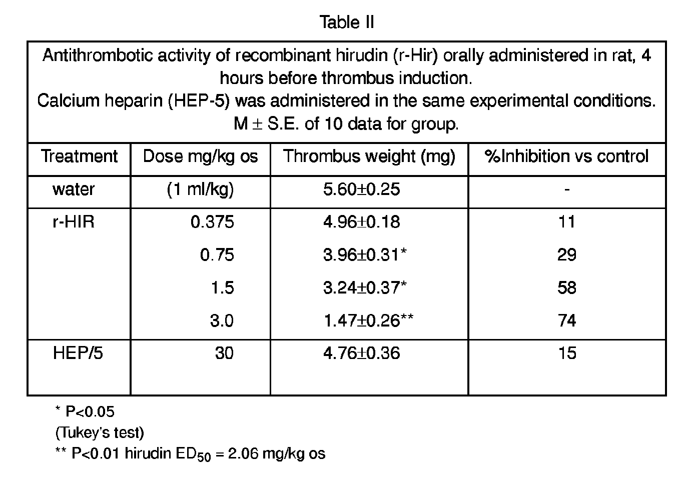

### Results and conclusions

Orally administered hirudin in rat showed a remarkable antithrombotic activity at 1.5 and 3 mg/kg doses; there is a trend towards the dose-effect relationship, in fact the regression was significant.

It is interesting to observe activity lenght: after four hours (through 0.5 and 4 hour observation), hirudin showed its most relevant activity.

Calcium heparin showed no anti-thrombotic effect at a extremely high dose (30 mg/kg os).

Blood withdrawals from abdominal aorta were carried out in sodium citrate, before removing thrombus (5 hours and 15 minutes after treatments), from 5 control rats and from 5 rats treated with hirudin (3 mg/kg os) and 5 rats treated with calcium heparin (30 mg/kg os). Plasma was separated and frozen to -30°C till the TT and PT determinations, which were executed by means of kits.

The examined substances, orally administered, caused no modifications of the considered coagulation parameters.

### EXAMPLE 3

Activity of recombinant hirudin (10,000 ATU/mg) microencapsulated in gastroresistant microbeads orally administered in the jugular vein thrombosis of rabbit.

The test was carried out under the same conditions of Example 1. Recombinant hirudin doses were 0.625, 1.25, 2.5 mg/kg. Results are summarized in Table III.

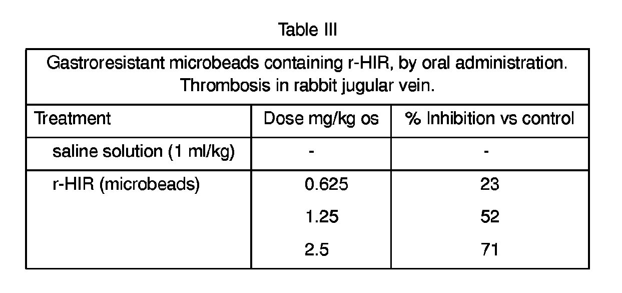

### EXAMPLE 4

Activity of recombinant hirudin (10,000 ATU/mg) "entrapped" in liposomes, by gastric probe administration.

Liposomes were prepared with well-known techniques; for example, by incorporating hirudin in egg lecithin with 10% stearyl amine or 10% ethyl phosphate and subjecting the mixture to sonication or phase reverse evaporation techniques. Liposome suspension was dispersed in cow milk, then administered with gastric probe. Thrombosis test in rat was carried out in the same way as in Example 2. Active ingredient doses were 0.3, 0.6, 1.2 mg/kg. Results are summarized in Table IV

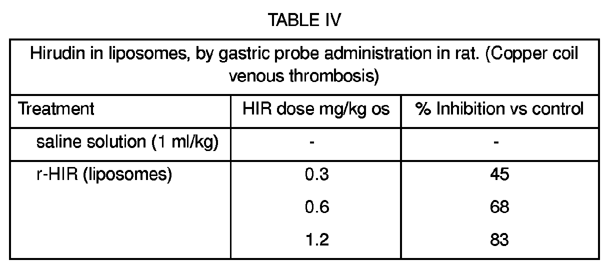

### EXAMPLE 5

Hard gelatin capsules containing recombinant hirudin.

Each capsule contains:

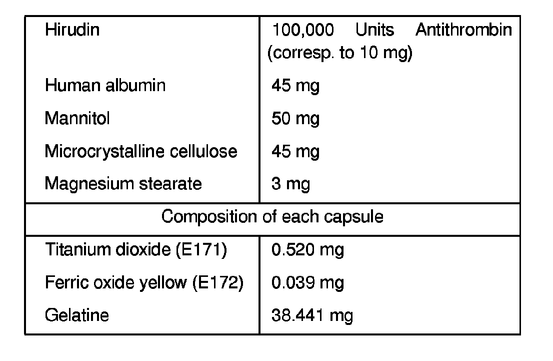

Capsules can be administered to humans either as such or coated with gastro-resistant materials, such as Eudragit.

## Claims (Claims for the following Contracting State(s) : AT, BE, CH, DE, DK, ES, FR, GB, IT, Ll, LU, NL, SE)

1. Recombinant hirudin as oral antithrombotic medicament.

2. Oral pharmaceutical compositions containing recombinant hirudin as the active principle in admixture with suitable excipients.

3. Oral pharmaceutical compositions according to claim 2 in the form of liposomial formulations.

4. The use of recombinant hirudin for the preparation of an oral anti-thrombotic medicament for the prevention of relapses of heart attacks.

## Claims (Claims for the following Contracting State(s) : ES, GR)

1. A process for the preparation of oral pharmaceutical compositions containing recombinant hirudin characterized in that said recombinant hirudin is admixed with one or more proper pharmaceutically acceptable vehicles fit for oral administration.

2. A process for the preparation of liposomial pharmaceutical compositions containing recombinant hirudin for oral administration characterized by the steps of incorporating recombinant hirudin in egg lecithin in the presence of stearyl amine or ethyl phosphate and of subjecting the mixture to sonication or phase reverse evaporation techniques.

## Patentansprüche (Patentansprüche für folgende(n) Vertragsstaat(en) : AT, BE, CH, DE, DK, FR, GB, IT, LI, LU, NL, SE)

1. Rekombinantes Hirudin als orales Antithrombose-Arzneimittel.

2. Orale pharmazeutische Zusammensetzungen, die rekombinantes Hirudin als Wirkstoff im Gemisch mit geeigneten Exzipienten enthalten.

3. Orale pharmazeutische Zusammensetzungen nach Anspruch 2 in Form von Liposom-Formulierungen.

4. Verwendung von rekombinantem Hirudin zur Herstellung eines oralen Antithrombose-Arzneimittels zur Verhinderung von Herzattacken-Rezidiven.

## Patentansprüche (Patentansprüche für folgende(n) Vertragsstaat(en) : ES, GR)

1. Verfahren zur Herstellung von oralen pharmazeutischen Zusammensetzungen, die rekombinantes Hirudin enthalten, dadurch gekennzeichnet, daß das rekombinante Hirudin mit einem oder mehreren geeigneten, pharmazeutisch akzeptablen Vehikula, die für die orale Verabreichung geeignet sind, gemischt wird.

2. Verfahren zur Herstellung von pharmazeutischen Liposom-Zusammensetzungen, die rekombinantes Hirudin enthalten, für die orale Verabreichung, gekennzeichnet durch die folgenden Stufen:
Einarbeitung von rekombinantem Hirudin in Ei-Lecithin in Gegenwart von Stearylamin oder Ethylphosphat und Behandeln der Mischung mit Ultraschall oder durch Anwendung von Phasenumkehr-Verdampfungsmethoden.

## Revendications (Revendications pour l'(les) Etat(s) contractant(s) suivant(s) : suivants : AT, BE, CH, DE, DK, FR, GB, IT, LI, LU, NL, SE)

1. Hirudine recombinante en tant que médicament antithrombotique à usage oral.

2. Compositions pharmaceutiques à usage oral contenant de l'hirudine recombinante en tant que principe actif en mélange avec des excipients appropriés.

3. Compositions pharmaceutiques à usage oral selon la revendication 2 sous la forme de formulation en liposomes.

4. Utilisation de l'hirudine recombinante pour la préparation d'un médicament anti-thrombotique à usage oral pour la prévention contre de nouvelles crises cardiaques.

## Revendications (Revendications pour l'(les) Etat(s) contractant(s) suivant(s) : suivants : ES, GR)

1. Procédé de préparation de compositions pharmaceutiques à usage oral contenant de l'hirudine recombinante, caractérisé en ce que ladite hirudine recombinante est mélangée avec un ou plusieurs véhicules pharmaceutique- ment acceptables appropriés adaptés à l'administration orale.

2. Procédé de préparation de compositions pharmaceutiques sous forme de liposomes, contenant de l'hirudine recombinante pour administration orale, caractérisé par les étapes consistant à incorporer de l'hirudine recombinante dans de la lécithine d'oeuf en présence d'amine stéarylique ou de phosphate d'éthyle et à soumettre le mélange à l'action des ultrasons ou à une évaporation avec inversion de phases.
